Europäisches Patentamt

European Patent Office

Office européen des brevets

(11) Veröffentlichungsnummer: **0 383 419**
**A1**

## (12) EUROPÄISCHE PATENTANMELDUNG

(21) Anmeldenummer: **90250045.3**

(22) Anmeldetag: **14.02.90**

(51) Int. Cl.5: **A61B 17/60**

(30) Priorität: **15.02.89 DE 8901908 U**

(43) Veröffentlichungstag der Anmeldung:
**22.08.90 Patentblatt 90/34**

(84) Benannte Vertragsstaaten:
**CH DE FR LI**

(71) Anmelder: **MECRON MEDIZINISCHE PRODUKTE GMBH**
**Nunsdorfer Ring 23-29**
**D-1000 Berlin 48(DE)**

(72) Erfinder: **Kranz, Curt, Dr. Ing.**
**Kufsteiner Strasse 12**
**D-1000 Berlin 62(DE)**
Erfinder: **Tetzner, Joachim, Dipl.-Ing.**
**Skarbinastrasse 78**
**D-1000 Berlin 49(DE)**

(74) Vertreter: **Christiansen, Henning, Dipl.-Ing.**
**Patentanwalt Pacelliallee 43/45**
**D-1000 Berlin 33(DE)**

(54) **Orthopädische Fixiereinrichtung.**

(57) Externe orthopädische Fixiereinrichtung mit mindestens zwei beidseitig eines Knochenbruches anzuordnenden, jeweils mindestens eine Knochenschraube oder einen Knochennagel aufnehmenden Basiselementen, welche über Gelenke und Klemmvorrichtungen mit stangenförmigen Bauteilen in Verbindung stehen, wobei mindestens ein Basiselement (4) über mindestens zwei Gelenke und eine oder mehrere Klemmvorrichtungen (5) mit einem stangenförmigen Bauteil (6) verbunden ist.

Fig.1

EP 0 383 419 A1

## Orthopädische Fixiereinrichtung

Die Erfindung betrifft eine externe orthopädische Fixiereinrichtung der im Oberbegriff des Anspruchs 1 angegebenen Art.

Orthopädische Fixiereinrichtungen dienen hauptsächlich der Stabilisierung infizierter Pseudarthrosen sowie der Versorgung offener Trümmerbrüche bei Menschen und Tieren.

Aus der DE-OS 27 45 504 ist eine Fixiervorrichtung bekannt, welche paarweise gegenüberliegende, Knochenschrauben oder Knochennägel aufnehmende Basiselemente aufweist, die über Klemmstücke mit stangenförmigen Bauteilen in Verbindung stehen und bei denen über Spanngeräte Distraktions- bzw. Kontraktionskräfte ausgeübt werden können, wobei die Einzelteile der Vorrichtung im wesentlichen aus faserverstärkten thermoplastischen Kunststoffen bestehen. Die Basiselemente sind an mindestens einer Seite über jeweils ein Kugelgelenk mit jeweils einem Spannstück verbunden. Eine derartige Fixiereinrichtung weist jedoch den Nachteil auf, daß bei seitlicher und axialer Belastung die Gefahr des Verrutschens der Klemmstücke auf den stangenförmigen Bauteilen sowie des Verdrehens der Kugelgelenke besteht.

Ferner geht aus der DE-OS 27 18 515 eine orthopädische Fixiereinrichtung hervor, bei der ein modifiziertes Kugelgelenk zur Anwendung kommt. Dieses bewirkt zwar eine erhöhte Festigkeit, die jedoch zur Überwindung der oben geschilderten Schwierigkeiten immer noch unzureichend ist.

Der Erfindung liegt die Aufgabe zugrunde, eine Fixiereinrichtung gemäß dem Oberbegriff des Anspruchs 1 derart weiterzubilden, daß deren Stabilität und Festigkeit den Anforderungen der Praxis mit hoher Sicherheit entsprechen. Insbesondere war die Erfindung auch auf die Überwindung der stabilitätsbedingten Notwendigkeit der gleichzeitigen Anwendung mehrerer Fixiereinrichtungen gerichtet.

Diese Aufgabe wird mit den kennzeichnenden Merkmalen des Anspruchs 1 gelöst. Vorteilhafte Weiterbildungen der Erfindung sind in den Unteransprüchen gekennzeichnet.

Erfindungsgemäß werden die Basiselemente an mindestens einer Seite über jeweils mindestens zwei Universalgelenke mit den Klemmvorrichtungen verbunden. Dadurch wird eine erhebliche Steigerung der Stabilität der Verbindung, vor allem im Hinblick auf Abrutsch- und Drehsicherheit erreicht. Die Erfindung beruht insbesondere auf der Erkenntnis, daß durch die Verdopplung der Zahl der Kugelgelenke im Bereich einer Klemmstelle durch das Vorsehen von zwei in einem Abstand zueinander angeordneter Kugelklemmverbindungen eine zusätzliche Sicherung durch Rotationsbewegungen gegeben ist, da diese - im Gegensatz zur Verwendung nur einer Kugel - durch die Lösung nach der Erfindung sicher gesperrt sind.

Entsprechend einer vorteilhaften Ausgestaltung der Erfindung sind die Universalgelenke als Kugelgelenke ausgebildet.

Bei Vorhandensein mehrerer stangenförmiger Bauteile, die verschiedene Basiselemente miteinander verbinden und annähernd parallel verlaufen, ist eine nochmalige Steigerung der Fixiersicherheit mit Hilfe mindestens eines Bügels zur zusätzlichen "Vernetzung" der stangenförmigen Bauteile möglich. Der Bügel ist mit seinen beiden Enden vorzugsweise über jeweils ein Kugelgelenk mit jeweils einem, dem stangenförmigen Bauteil zugeordneten Basiselement verbunden. Die Kupplungsstelle befindet sich dabei an der zur Längsrichtung eines stangenförmigen Bauteiles senkrechten oder nahezu senkrechten Stirnfläche des Basiselementes und ist vorzugsweise als Kugel ausgebildet, welche einen Teil des Kugelgelenkes bildet.

Damit die röntgenologische Untersuchung der Bruchzone nicht behindert wird und um möglichst geringes Gewicht zu erreichen, wird metallisches Material weitgehend vermieden. Die Basiselemente und die Klemmeinrichtungen bestehen daher im wesentlichen aus Kunststoff und die stangenförmigen Bauteile sind als Kohlefaserrohre ausgebildet.

Bei der erfindungsgemäßen Vorrichtung zur Ruhigstellung oder Stützung von Gliedmaßen von Menschen und Tieren sind paarweise gegenüberliegende Knochenschrauben oder Knochennägel aufnehmende Verbindungsteile vorgesehen, die über Klemmelemente mit Spannschlößern bzw. Gewindestäben in Verbindung stehen und bei denen über Spanngeräte Distraktions- bzw. Kompressionskräfte - z.B. in einer Bruchzone - ausgeübt werden können. Die Einzelteile dieser Vorrichtungen - wie Kupplungsstücke, Rundstäbe, Leisten usw. - bestehen im wesentlichen aus Metall.

Der Schwerpunkt ihrer Anwendung liegt heute bei der Stabilisierung infizierter Pseudarthrosen, bei der Versorgung offener Trümmerbrüche - insbesondere der unteren Gliedmaßen - sowie bei Arthrodesen großer Gelenke. Als Nachteil aller bisher bekannten äußeren Knochenverankerungen ist anzusehen, daß sie aus Metall gefertigt sind und damit -insbesonders bei räumlicher Anordnung - eine einwandfreie röntgenologische Beurteilung der Bruchzone erheblich erschwert bzw. unmöglich wird. Fehlstellungen am Knochen sind daher häufig nicht zu erkennen und auch nicht zu beheben, es sei denn, man fertigt zusätzlich Schrägaufnahmen an oder entfernt vor dem Röntgen die äußere Knochenverankerung. Letzteres ist jedoch mit einer vorübergehenden In stabilität in der Knochenbruchzone verbunden. Insbesondere bei den aufwendigen räumlichen Anordnungen klagen die Patienten häufig über deren hohes Gewicht. Die Folge ist eine reduzierte Mobilität der Patienten mit entsprechenden Auswirkungen auf ihre psychische Gesamtsituation und auf die Kreislaufverhältnisse an den verletzten Gliedmaßen mit der bekannten Gefahr von Blutabflußstörungen.

Bei der erfindungsgemäßen Vorrichtung sind wenige unterschiedlich gestaltete Verbindungsteile zur Lösung einer Vielzahl von medizinischen Aufgabenstellungen im Osteosynthesebereich geeignet, wobei die Verbindungsteile, die Klemmstücke und die Verbindungsstäbe aus thermoplastischen Kunststoffen bestehen. Die Vorrichtung zeichnet sich durch hohe Stabilität, Sterilisierbarkeit und gute Wärmeformbeständigkeit aus. Darüberhinaus sind die Elemente röntgenstrahlentransparent sowie von körperfreundlicher Gestalt. Sie erlauben eine einfache Handhabung und wirtschaftliche Herstellung. Mit nur vier Grundelementen ergibt sich eine Vielzahl von Montagevarianten. Von herausragendem Vorteil ist schließlich eine deutliche Gewichtsreduzierung.

Beispielhafte Ausführungsformen der erfindungsgemäßen Fixiereinrichtung sowie vergrößerte Details sind in den Zeichnungen schematisch dargestellt und im Folgenden näher erläutert. Es zeigen:

Figur 1 eine Ausführungsform der erfindungsgemäßen Fixiereinrichtung in perspektivischer Darstellung,

Figur 2 eine Draufsicht auf ein Basiselement gemäß Figur 1,

Figur 3 eine weitere Ausführungsform der erfindungsgemäßen Fixiereinrichtung,

Figur 4 eine Draufsicht auf ein Basiselement gemäß Figur 3,

Figur 5 einen Längsschnitt durch eine externe, abnehmbare Klemmvorrichtung und

Figur 6 schematisch eine integrierte Spannvorrichtung.

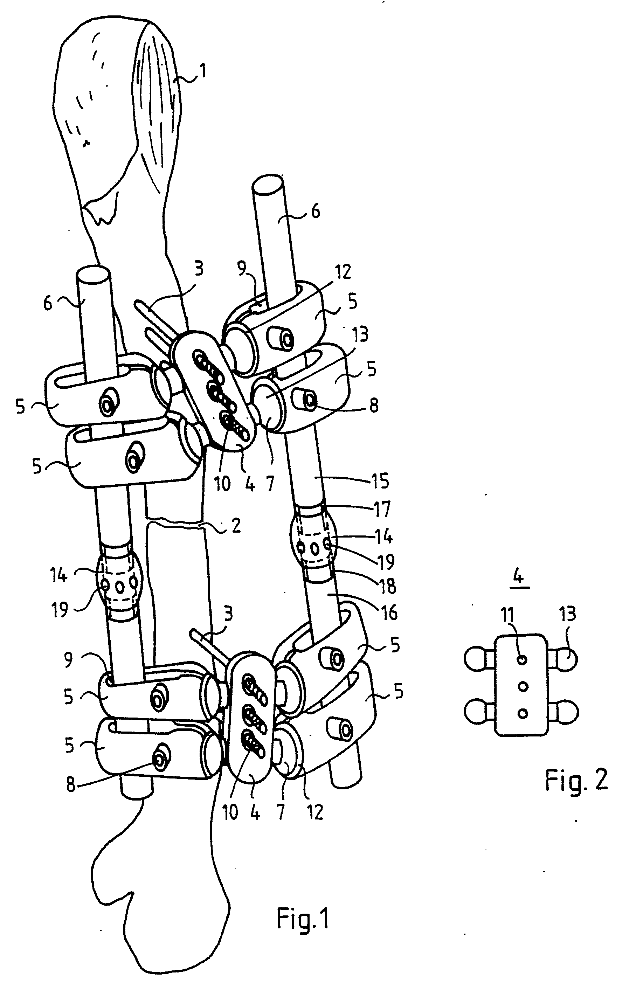

In Figur 1 bedeutet 1 den zu versorgenden Knochen mit dem Bruchspalt 2. Beiderseits des Bruchspaltes 2 werden die Knochenschrauben 3 durch die Basiselemente 4 in den Knochen 1 eingeschraubt. Die beiden Basiselemente 4 werden über acht Klemmvorrichtungen 5 mit zwei stangenförmigen Bauteilen 6 derart verbunden, daß ein stabiler äußerer Rahmen entsteht. Dabei werden die Enden der stangenförmigen Bauteile 6 von jeweils zwei Klemmvorrichtungen 5 gehalten, wobei diese beiden Klemmvorrichtungen 5 über zwei Kugelgelenke 7 mit einer Seitenfläche eines Basiselementes 4 verbunden sind. Im nicht fixierten Zustand sind die Grundelemente gegeneinander räumlich bewegbar. Zur Fixierung der gewünschten Stellung dienen die an den Klemmvorrichtungen 5 angebrachten Innensechskantschrauben 8. Um den Basiselementen 4 einen gewissen Verdrehungsspielraum gegenüber der Erstreckungsrichtung des Knochen 1 und gegenüber den stangenförmigen Bauteilen 6 zu ermöglichen, sind die Klemmvorrichtungen 5 mit insbesondere die Kontour von Langlöchern aufweisenden Backen 9 zur Aufnahme der stangenförmigen Bauteile 6 versehen.

Zur Erreichung eines vorgespannten Formschlusses zwischen den Knochenschrauben 3 und den Basiselementen 4, weisen die Knochenschrauben 3 an den gewindefreien Teilen eine Kreuzrändelung 10 auf, sodaß sie sich aufgrund örtlicher plastischer Verformung in der Bohrung 11 (Fig. 2) des Basiselementes 4 verkrallen. Eine zusätzliche Feststellschraube ist daher nicht notwendig.

Die Klemmvorrichtungen 5 sind mit elastisch aufweitbaren Kugelpfannen 12 ausgestattet. Damit ist gewährleistet, daß die Klemmvorrichtungen 5 mit geringem Kraftaufwand auf die Gelenkkugeln 13 der Basiselemente 4 aufsetzbar sind. Die Gleitflächen der Gelenkkugeln 13 der Basiselemente 4 sind mit Quarzmehl beschichtet, wodurch ohne zusätzliche Vorkehrungen eine sichere Haftung zwischen den Basiselementen und den Klemmvorrichtungen erzielbar ist.

Zuvor werden die stangenförmigen Bauteile 6 der den speziellen Bedingungen des Bruches entsprechenden Länge in die Backen 9 der Klemmvorrichtungen 5 eingeschoben. Die endgültig gewählte Stellung von Basiselementen 4, Klemmvorrichtungen 5 und stangenförmigen Bauteilen 6 zueinander wird mit den Innensechskantschrauben 8 kraftschlüssig gesichert.

Die erfindungsgemäße Fixiereinrichtung kann auch mit integrierten Spanneinrichtungen versehen sein. Diese besteht aus einem Spannschloß mit einem Rechts- und einem Linksinnengewinde, in welchen die beiden Teile 15 und 16 des stangenförmigen Bauteiles 6 mit den entsprechenden Außengewinden 17 und 18 eingeschraubt werden. Distraktion und Kompression werden durch Verdrehen des Spannschlosses 14 bewirkt. Besonders hohe Kompressionskräfte lassen sich über einen Verdrehstift aufbringen, der in entsprechende Aufnahmebohrungen 19 des Spannschlosses 14 einsetzbar ist.

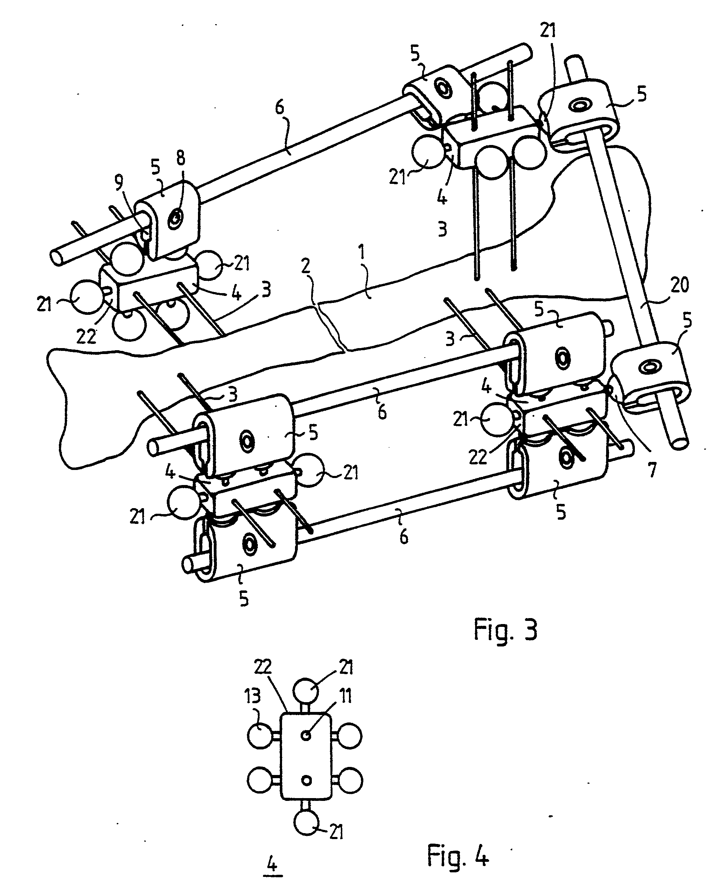

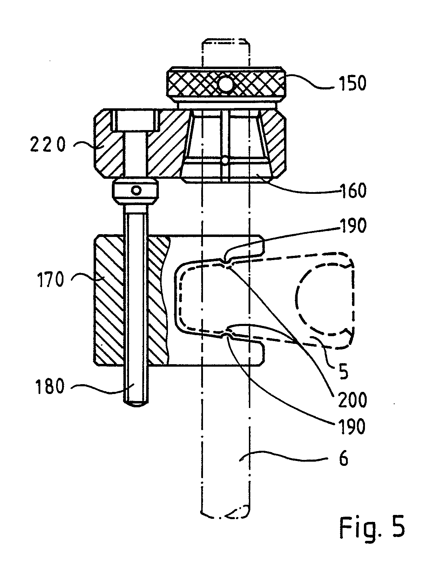

In Figur 3 ist eine gegenüber der in Figur 1 dargestellten Variante erweiterte Ausführungsform der erfindungsgemäßen Fixiereinrichtung gezeigt. Die Stabilität dieser Ausführungsform ist durch die Erhöhung der Anzahl der Basiselemente von zwei auf vier und durch zwei Querverbindungen zwischen den jeweils zwei annähernd konzentrisch um den Knochen angeordneten Basiselementen noch einmal erhöht worden. Diese Querverbindungen in Form von Bügeln 20 sind ebenfalls als Kugelgelenke 7 ausgebildet, wobei die Kugeln 21, wie in Figur 4 dargestellt, an den zur Erstreckungsrichtung der stangenförmigen Bauteile 6 senkrechten oder nahezu senkrechten Stirnflächen 22 der Basiselemente 4 vorgesehen sind.

Aus röntgentechnischen und aus Gewichtsgründen bestehen die Klemmvorrichtungen 5 und die Basiselemente 4 im wesentlichen aus Kunststoff, während die stangenförmigen Bauteile 6 als Kohlefaserrohre ausgebildet sind.

Figur 5 zeigt die externe - abnehmbare - Spannvorrichtung für Distraktion und Kompression, die zur Ausübung dieser Kräfte auf die freistehenden Enden der in Figur 1 gezeigten Verbindungsstangen parallel zur Verstellrichtung stirnseitig aufgeschoben und mit der Feststellmutter 150, sowie dem geteilten Konusstück 160 fixiert wird. Die Mitnahmegabel 170 wird auf das Klemmstück 5 eingeschwenkt.

Der Nocken 190 am Teil 170 und die Nuten 200 im Klemmstück 5 verhindern ein Abgleiten der Spannvorrichtung. Durch Verdrehen des Gewindestabes 180 wird der Abstand zwischen dem Aufnahmeteil 220 des Konusstücks und der Mitnahmegabel 170 verringert bzw. vergrößert. Distraktion respektive Kompression können so ausgeübt werden. Diese externe Spannvorrichtung kann aus thermoplastischen Kunststoffen oder auch aus Metall gefertigt sein.

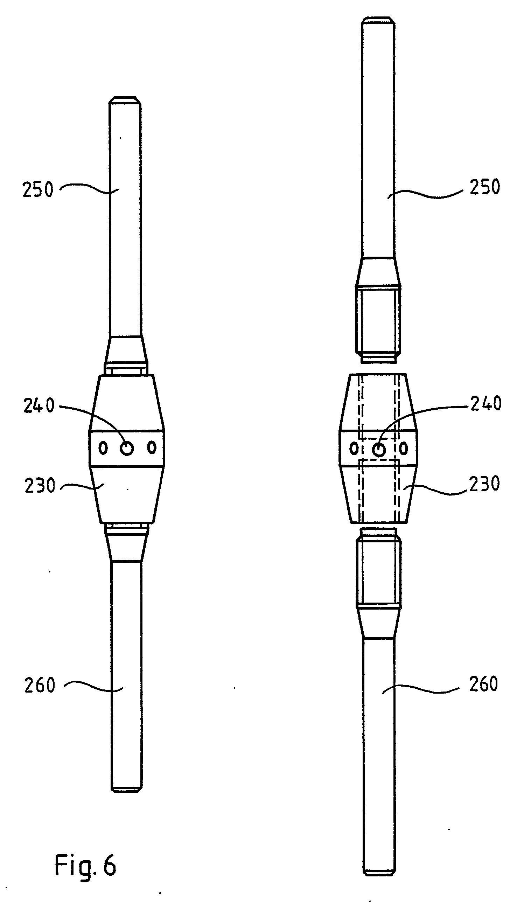

Die erfindungsgemäße Vorrichtung kann auch mit integrierten Spanneinrichtungen versehen sein, die anstelle des Verbindungsstabes 6 der äußeren Knochenverankerung in das Klemmstück 5 eingeschoben werden. Wie Figur 6 zeigt, bestehen sie aus einem Spannschloß 230 mit einem Rechts- und einem Linksinnengewinde, in das die Stäbe 250 und 260 mit entsprechenden Außengewinden eingeschraubt werden. Distraktion und Kompression werden durch Verdrehen des Spannschloßes von Hand bewirkt. Besonders hohe Kompressionskräfte werden über einen Verdrehstift aufgebracht, der in entsprechenden Aufnahmebohrungen 240 des Spannschloßes eingerastet wird.

Anstelle der Verbindungsrundstäbe 6 sind auch bandförmige Elemente, Halbschalen oder Mantelrohre denkbar. Ihre Anwendung ist jedoch vielfach nur in Sonderfällen zweckmäßig. Der damit mögliche Stabilitätsgewinn ist nur durch das Einbringen weiterer Knochenschrauben zu realisieren, führt jedoch zu einer Reduzierung der Zahl möglicher Montagevarianten.

Die Erfindung beschränkt sich in ihrer Ausführung nicht auf das vorstehend angegebene bevorzugte Ausführungsbei spiel. Vielmehr ist eine Anzahl von Varianten denkbar, welche von der dargestellten Lösung auch bei grundsätzlich anders gearteten Ausführungen Gebrauch machen.

## Ansprüche

1. Externe orthopädische Fixiereinrichtung mit mindestens zwei beidseitig eines Knochenbruches anzuordnenden, jeweils mindestens eine Knochenschraube oder einen Knochennagel aufnehmenden Basiselementen, welche über Gelenke und Klemmvorrichtungen mit stangenförmigen Bauteilen in Verbindung stehen, **dadurch gekennzeichnet,** daß mindestens ein Basiselement (4) über mindestens zwei Gelenke und eine oder mehrere Klemmvorrichtungen (5) mit einem stangenförmigen Bauteil (6) verbunden ist.

2. Fixiereinrichtung nach Anspruch 1, **dadurch gekennzeichnet,** daß die Gelenke als Kugelgelenke (7) ausgebildet sind.

3. Fixiereinrichtung nach einem der vorhergehenden Ansprüche, **dadurch gekennzeichnet,** daß mindestens ein Bügel (20) zur Verbindung mindestens zweier stangenförmiger Bauteile (6) vorgesehen ist.

4. Fixiereinrichtung nach Anspruch 3, **dadurch gekennzeichnet,** daß der Bügel (20) an beiden Enden über jeweils ein Kugelgelenk (7) mit jeweils ei nem, dem stangenförmigen Bauteil (6) zugeordneten Basiselement (4) verbunden ist.

5. Fixiereinrichtung nach Anspruch 4, **dadurch gekennzeichnet,** daß eine Kugel (21) an der zur Längsrichtung eines stangenförmigen Bauteiles (6) senkrechten oder nahezu senkrechten Stirnfläche (22) des Basiselementes (4) einen Teil des Kugelgelenkes (7) bildet.

6. Fixiereinrichtung nach einem der vorhergehenden Ansprüche, **dadurch gekennzeichnet,** daß die Klemmvorrichtungen (5) und die Basiselemente (4) im wesentlichen aus Kunststoff bestehen.

7. Fixiereinrichtung nach einem der vorhergehenden Ansprüche, **dadurch gekennzeichnet,** daß die stangenförmigen Bauteile (6) als Kohlefaserrohre ausgebildet sind.

8. Fixiereinrichtung nach einem der vorangehenden Ansprüche, **dadurch gekennzeichnet,** daß mindestens ein Spannschloss (230) in einen Verbindungsstab (6) integriert ist.

9. Fixiereinrichtung nach einem der vorangehenden Ansprüche, **dadurch gekennzeichnet,** daß eine verstellbare Spanneinrichtung vorgesehen ist, welche ein Verbindungselement (160) zum festen Verbinden mit einem stangenförmiges Bauteil (6) sowie eine über eine Gelenkkugel schwenkbare diese beidseitig begrenzende Gabel (170) aufweist, wobei Verbindungselement und Gabel durch ein Verstellelement (180) relativ zueinander verschiebbar sind.

ME 39.2-EU

Fig.1

Fig. 2

ME 39.2-EU

Fig. 3

4

Fig. 4

ME 39.2 - EU

150

220

160

190

170

5

200

190

180

6

Fig. 5

ME 39.2-EU

250

240

230

260

250

240

230

260

Fig. 6

Europäisches
Patentamt

**EUROPÄISCHER RECHERCHENBERICHT**

Nummer der Anmeldung

EP 90 25 0045

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | FR-A-2 405 063 (KNOLL)<br>* Insgesamt * | 1-2,6,8<br>-9 | A 61 B 17/60 |
| Y,D | | 3-5,7 | |
| | --- | | |
| X | FR-A-2 393 563 (J.-C. FIORDARANCIO)<br>* Seite 1, Zeilen 4-21; Seite 2, Zeilen 7-23,30-33; Figuren 1,2 * | 1-2 | |
| | --- | | |
| X | US-A-4 696 293 (J.V. CIULLO)<br>* Spalte 2, Zeile 64 - Spalte 3, Zeile 3; Spalte 4, Zeile 1-7,26-32; Spalte 5, Zeilen 20-29; Figur 1 * | 1,8 | |
| | --- | | |
| Y | DE-A-2 928 216 (POLDI SPOJENE OCELARNY)<br>* Seite 9, Zeile 18 - Seite 10, Zeile 6; Figur 7 * | 3-5 | |
| | --- | | |
| Y | MEDIZINISCH-ORTHOPÄDISCHE TECNIK, Band 103, Nr. 3, Mai/Juni 1983, Seiten 79-82, Stuttgart, DE; Th. STUHLER: "Katastrophensituationen: Variationsmöglichkeiten eines Fixateur externe"<br>* Seite 81, linke Spalte: "Führungsstangen * | 7 | |
| | ------ | | |

|  |  |
|---|---|
|  | **RECHERCHIERTE SACHGEBIETE (Int. Cl.5)**<br><br>A 61 B<br>A 61 F |

Der vorliegende Recherchenbericht wurde für alle Patentansprüche erstellt

| Recherchenort | Abschlußdatum der Recherche | Prüfer |
|---|---|---|
| DEN HAAG | 15-05-1990 | NICE P.R. |